# EUROPEAN PATENT APPLICATION

(11) **EP 4 591 795 A1**
(43) Date of publication of application: **30.07.2025**
(21) Application number: 24213151.4
(22) Date of filing: 15.11.2024
(51) Int. Cl.: A61B 5/367, A61B 5/00

(54) **GUI TO DISPLAY LOCATIONS OF EARLY AND LATE LOCAL ACTIVATION TIMES (LAT) IN LAT MAP AND CORRECT THE MAP**

(30) Priority: 29.01.2024 US 202418424921
(71) Applicant: Biosense Webster (Israel) Ltd., 2066717 Yokneam (IL)
(72) Inventor: GOVARI, Assaf, 2066717 Yokneam (IL); GLINER, Vadim, 2066717 Yokneam (IL)
(74) Representative: Carpmaels & Ransford LLP

(57) **Abstract**

A system includes a display device, an input device, and a processor. The processor is configured to (i) receive a set of data points representing a full range of the EP parameter, (ii) responsively to one or more pre-defined percentile ranges, generate a partial-data EP map using only data points belonging to the one or more predefined percentile ranges, (iii) display the partial-data EP map to a user on the display device, (iv) receive from the user, via the input device, a selection on the partial-data EP map of one or more outlier data points belonging to one or more given percentile ranges, (v) regenerate the EP map using the set of data points without the selected outlier data points, and (vi) display the regenerated EP map to the user.

## Description

### FIELD OF THE DISCLOSURE

The present invention relates generally to electrophysiological mapping, and particularly to visualization of cardiac electrophysiological data points and maps.

### BACKGROUND OF THE DISCLOSURE

An electrophysiological (EP) map of a cardiac chamber of a patient is generated by positioning electrodes on a region of the chamber's tissue, acquiring an EP signal of the region, and then repeating the process for a different region. EP parameters are extracted from the EP signals in each region of measurement, and then displayed over a graphical representation of the tissue, such as a three-dimensional (3D) rendering of the cardiac chamber.

EP mapping visualization methods previously proposed in the patent literature, to ease an interpretation of an EP map. For example, U.S. Patent Application Publication 2022/0338783 describes a method including receiving a plurality of data points including electrophysiological (EP) values measured at respective positions in at least a portion of an organ of a patient. Some of the EP values are classified as outlier values in accordance with a defined criterion. A visual representation of at least the portion of the organ is derived from the plurality of data points. The visual representation represents the EP values with respective colors, and visualizes less than all the outlier values, by performing one or both of (a) identifying outlier values that deviate from respective neighboring EP values by less than a defined deviation, and representing the outlier values using colors that match the neighboring EP values, and (b) setting for the visual representation a mapping, which maps the EP values to the colors and which excludes at least some of the outlier values.

As another example, U.S. Patent Application Publication 2022/0095942 describes a medical apparatus that includes a probe configured for insertion into a body of a patient. The probe includes electrodes configured to contact tissue of a region within the body. The apparatus further includes a display screen a position-tracking system, and a processor. The processor is configured to acquire electrophysiological signals from the electrodes, to extract electrophysiological parameters from the signals, to compute a measure of consistency of the electrophysiological parameters at each of the locations with respect to a weighted median of the parameters extracted at neighboring locations, and to render to the display screen a map of the tissue while overlaying on the map a visual indication of the extracted electrophysiological parameters for which the measure of consistency satisfied a predefined consistency criterion, and automatically omitting from the map the electrophysiological parameters for which the measure of consistency did not satisfy the criterion.

The present disclosure will be more fully understood from the following detailed description of the examples thereof, taken together with the drawings, in which:

### BRIEF DESCRIPTION OF THE DRAWINGS

Fig. 1 is a schematic, pictorial illustration of a catheter-based electrophysiological (EP) mapping and ablation system, in accordance with an example of the present disclosure;
Fig. 2 is a schematic, pictorial volume rendering of an LAT map of a cardiac chamber including highlighted outlier data points, in accordance with an example of the present disclosure;
Fig. 3 is a flow chart that schematically illustrates a method for using a GUI and an input device to correct an LAT map by removing outlier data points, in accordance with an example of the present disclosure; and
Fig. 4 is a flow chart that schematically illustrates a method for using a GUI and an input device to correct an LAT map by removing outlier data points, in accordance with another example of the present disclosure.

### DETAILED DESCRIPTION OF EXAMPLES

### OVERVIEW

During an EP mapping procedure, an EP map, such as a local activation time (LAT) map, is often generated based on EP data captured at a plurality of locations inside a cardiac chamber. A LAT map is a useful diagnostic tool when mapping during PVC (premature ventricular contraction), atrial flutter, WPW (Wolff-Parkinson-White syndrome), AVRT (atrioventricular re-entry tachycardia) and AVNRT (atrioventricular node re-entry tachycardia).

To generate an LAT map of the cardiac tissue of a patient (also called "electroanatomical mapping"), a physician positions a probe such that its electrodes contact locations at a region of the tissue. The probe acquires EP signals from the respective locations, and the process is then repeated over other regions. A processor analyzes the signal from each location in the region where it is acquired and extracts its LAT value. The LAT values are then overlaid on a visual 3D map of the region, for example as a color code, to form an LAT map that can be viewed by the physician. Such mapping may be performed in real time.

In a typical LAT mapping procedure, one catheter senses the activation wave (reference signal) while a mapping catheter measures the resulting activations (substrate signal). A processor annotates the reference signal and the respective substrate activation. Typically, hundreds of waveforms are acquired and annotated during the mapping procedure. Using the annotations, the processor calculates the respective LAT value between the reference signal and the respective substrate signal.

LAT values largely vary between a minimal value and a maximal value according to substrate tissue location in a cardiac chamber. However, some wrong LAT values occur due to incorrect annotation, typically of the more complex substrate signals. These values are called "outliers" or "outlying values." For example, incorrect annotation occurs when the reference signal or the substrate signal is fractionated. The outliers that appear at the tail end of the portion of the heart cycle (i.e., within a given percentile of the shortest LAT values, and a given percentile of the longest LAT values) cause the most significant errors to the colored map that you create, e.g., by distorting the color scale of the LAT map.

Region-wise, at some locations in a cardiac tissue region, the extracted LAT values may not be consistent with the LAT values at neighboring locations. Such outliers may be due to, for example, a fractionated EP signal at a certain location in the region that causes an incorrect (automatic) annotation.

As noted above, an outlier may have a significant effect on the visualization (e.g., coloring) of an LAT map, especially when the outlier point is in an area with a sparse amount of data. In such a case, the LAT map would be misleading (e.g., with a wrong scale). By skewing the LAT map visually, some outliers may provide the physician with incorrect information regarding the electrical propagation in the cardiac chamber.

The disclosed technique relies on the inventors' observation that outlier EP parameter data points (e.g., LAT) with a very low or a very high EP parameter value (within the full range of measured EP parameter values) cause the largest distortion to the LAT map (e.g., by skewing EP activity color coding). In the remainder of this disclosure, these outliers are often called "most significant outliers."

One example of the present disclosure that is described herein makes available a system including a processor configured to provide, using a display device and an input device (e.g., one comprising a touchscreen or a computer mouse) also provided, a graphical user interface (GUI) feature (e.g., a rolling scale) that lets a user define one or more percentile ranges of an Electrophysiological (EP) parameter. The processor receives a set of data points representing a full range of the EP parameter. Responsively to the one or more percentile ranges defined by the user, the processor generates a partial-data EP map using only data points belonging to the one or more percentile ranges. The processor displays the partial-data EP map to a user on the display device, and thereafter receives from the user, via the input device, a selection on the partial-data EP map of one or more outlier data points belonging to one or more given percentile ranges.

For example, the EP parameter (e.g., LAT) values may belong to a given highest percentile and lowest percentile of a full data set. Specifically to the example, the LAT values displayed may belong to the percentile ranges below 10% (i.e., 10% lowest values) and above 90% (e.g., 10% highest values.

Since only a limited cohort of data points is displayed using the above techniques, it is simpler for the user to identify and remove (e.g., delete from the data set) the most significant outliers. Consequently, the user may generate a corrected LAT map that is not distorted by the most significant outliers.

In another example, the processor provides, using the display device and the input device, a GUI feature that lets a user define one or more percentile ranges of the EP parameter within a full range of the given EP parameter. The processor presents, on the display device, an EP map having the full range of the EP parameter values. Responsively to the one or more percentile ranges defined by the user, the processor highlights on the EP map data points belonging to the one or more given percentile ranges. Thereafter, the processor receives from the user, via the input device, a selection on the highlighted EP map of one or more outlier data points belonging to one or more given percentile ranges.

In this example, the data points with LAT values belonging to the high/low percentile may be highlighted. Moreover, the high LAT value data points and the low LAT value data points may be highlighted differently, which may further ease the work of the user.

The system of the above example allows the user, using the input device, to remove the selected outlier data points. After the selected outlier data points are removed from the set, the processor automatically regenerates an EP map based on the "outlier cleaned" set of data points.

### SYSTEM DESCRIPTION

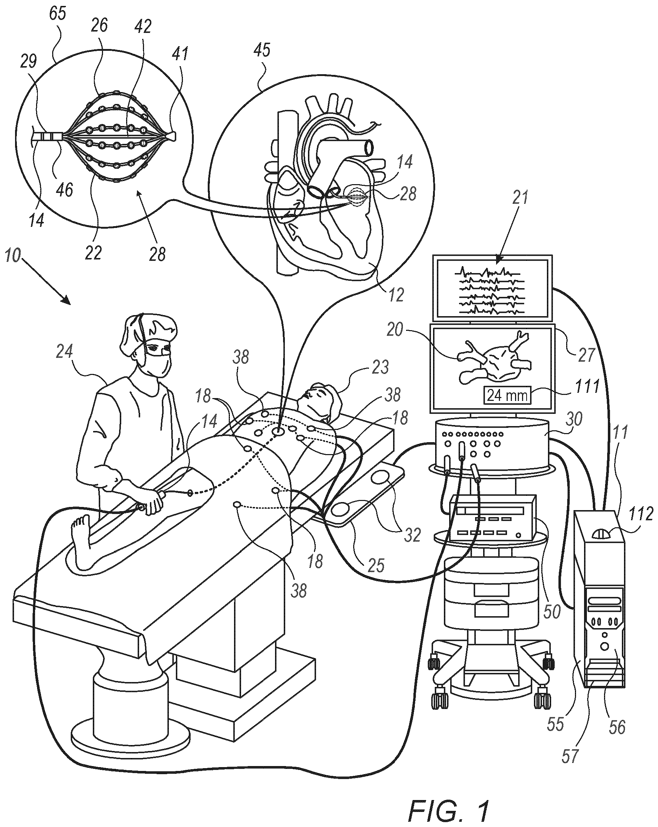

Fig. 1 is a schematic, pictorial illustration of a catheter-based electrophysiological (EP) mapping and ablation system 10, in accordance with an example of the present disclosure.

System 10 includes multiple catheters which are percutaneously inserted by physician 24 through the patient's vascular system into a chamber or vascular structure of a heart 12 (seen in inset 45). Typically, a delivery sheath catheter is inserted into a cardiac chamber, such as the left or right atrium near a desired location in heart 12. Thereafter, a plurality of catheters is inserted into the delivery sheath catheter in order to arrive at the desired location. The plurality of catheters may include a catheter dedicated for pacing, a catheter for sensing intracardiac electrogram signals, a catheter dedicated for ablating and/or a catheter dedicated for both EP mapping and ablating. An example catheter 14, illustrated herein, is configured for sensing bipolar electrograms. Physician 24 brings a distal tip 28 (also called hereinafter distal end assembly 28) of catheter 14 into contact with the heart wall for sensing a target site in heart 12. For ablation, physician 24 similarly brings a distal end of an ablation catheter to a target site.

As seen in inset 65, catheter 14 is an exemplary catheter that includes a basket distal end 28, including one, and preferably multiple, electrodes 26 optionally distributed over a plurality of splines 22 at distal tip 28 and configured to sense IEGM signals. Catheter 14 may additionally include a position sensor 29 embedded in or near distal tip 28 on a shaft 46 of catheter 14, used to track the position and orientation of distal tip 28. Optionally, and preferably, position sensor 29 is a magnetic-based position sensor including three magnetic coils for sensing three-dimensional (3D) position and orientation. As seen, distal tip 28 further includes an expansion/collapse rod 42 of expandable assembly 28 that is mechanically connected to basket assembly 28 at a distal edge 41 of assembly 28.

Magnetic based position sensor 29 may be operated together with a location pad 25 that includes a plurality of magnetic coils 32 configured to generate magnetic fields in a predefined working volume. Real-time position of distal tip 28 of catheter 14 may be tracked based on magnetic fields generated with location pad 25 and sensed by magnetic based position sensor 29. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091.

System 10 includes one or more electrode patches 38 positioned for skin contact on patient 23 to establish a location reference for location pad 25 as well as impedance-based tracking of electrodes 26. For impedance-based tracking, electrical current is directed toward electrodes 26 and sensed at electrode skin patches 38 so that the location of each electrode can be triangulated via electrode patches 38. Details of the impedance-based location tracking technology are described in US Patent Nos. 7,536,218; 7,756,576; 7,848,787; 7,869,865; and 8,456,182.

A recorder 11 displays, on display device 27, cardiac signals 21 (e.g., electrograms acquired at respectively tracked cardiac tissue positions) acquired with body surface ECG electrodes 18 and intracardiac electrograms acquired with electrodes 26 of catheter 14. Recorder 11 may include pacing capability to pace the heart rhythm, and/or may be electrically connected to a standalone pacer.

Workstation 55 includes memory 57, a processor 56 unit with memory or storage with appropriate operating software loaded therein, and user interface capability. Workstation 55 may provide multiple functions, optionally including (i) modeling endocardial anatomy in three-dimensions (3D) and rendering the model or EP map 20 for display on display device 27, (ii) displaying on display device 27 activation sequences (or other data) compiled from recorded cardiac signals 21 in representative visual indicia or imagery superimposed on the rendered EP map 20, (iii) displaying real-time location and orientation of multiple catheters within the heart chamber, and (iv) displaying sites of interest on display device 27, such as places where ablation energy has been applied. One commercial product embodying elements of system 10 is available as the CARTO^{™} 3 System, available from Biosense Webster, Inc., 31A Technology Drive, Irvine, CA 92618.

In a disclosed example, physician 23 uses a GUI 111 to generate the above-mentioned partial-data EP map (e.g., partial-data LAT map) to the percentile of EP parameter values, or to highlight portions of an EP map 20 according to the percentile of EP parameter values.

In the first case, physician 24 uses GUI 111 to select one or more given percentile ranges of given parameter EP values among the full range of the given EP parameter values and to command the generation of a partial-data EP map using only data points belonging to one or more given percentile ranges. Processor 56 receives a set of data points representing a given full range of the EP parameter values. Upon receiving the command from the user via GUI 111, processor 56 generates the partial-data EP map and displays the partial-data EP map to a user on a display device 27. An input device (e.g., one comprising a touchscreen 27 or computer mouse 112) is configured to let physician 23 select one or more outlier data points on the partial-data EP map that belong to one or more given percentile ranges. GUI 111 is further configured to allow physician 24 to regenerate the EP map based on the set of data points after the selected outlier data points are removed from the set.

As another example, physician 24 uses GUI 111 to select one or more given percentile ranges of given parameter EP values among the full range of the given EP parameter values and to command the highlighting of data points, on an EP map, belonging to the one or more given percentile ranges. Processor 56 is configured to (i) present an EP map having a given full range of the EP parameter values on display device 27, and (ii) highlight the data points, on the EP map, belonging to the one or more given percentile ranges upon receiving the command from physician 24 via GUI 111. The input device is configured to let physician 24 select one or more outlier data points on the highlighted LAT map that belong to one or more given percentile ranges. Using the input device (27, 112), the physician may remove some outliers and regenerate a corrected map.

System 10 may include an ablation energy generator 50 that is adapted to conduct ablative energy to one or more electrodes at a distal tip of a catheter configured for ablation. Energy produced by ablation energy generator 50 may include, but is not limited to, radiofrequency (RF) energy or pulsed-field ablation (PFA) energy, including monopolar or bipolar high-voltage DC pulses, to be used to effect irreversible electroporation (IRE), or combinations thereof.

Patient interface unit (PIU) 30 is an interface configured to establish electrical communication between catheters, electrophysiological equipment, power supply and a workstation 55 to control system 10 operation and to receive EA signals from the catheter. Electrophysiological equipment of system 10 may include, for example, multiple catheters, location pad 25, body surface ECG electrodes 18, electrode patches 38, ablation energy generator 50, and recorder 11. Optionally, and preferably, PIU 30 additionally includes processing capability for implementing real-time computations of catheter locations and for performing ECG calculations.

In some examples, processor 56 typically comprises a general-purpose computer, which is programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

This configuration of system 10 is shown by way of example, to illustrate certain problems that are addressed by examples of the present disclosure and to demonstrate the application of these examples in enhancing the performance of such a system. Examples of the present disclosure, however, are by no means limited to this specific sort of example system, and the principles described herein may similarly be applied to other types of medical systems. For example, other multi-electrode catheter types may be used, such as the multi-arm OCTARAY^{™} catheter or a flat catheter.

### OUTLIER DATA POINTS OF EARLY AND LATE LOCAL ACTIVATION TIMES

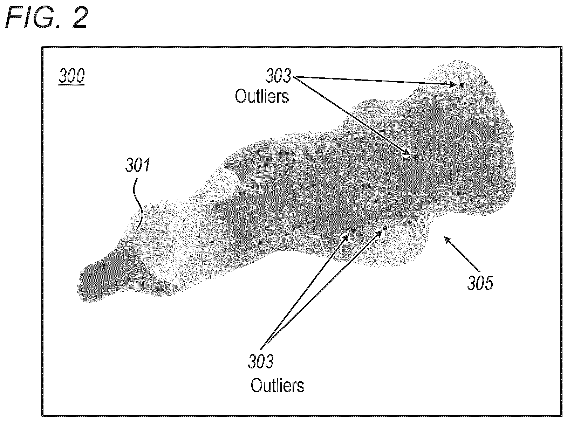

Fig. 2 is a schematic, pictorial volume rendering 300 of a local activation time (LAT) map 301 of a cardiac chamber including highlighted outlier data points (303), in accordance with an example of the present disclosure. While map 301 is mostly generated from a majority of welldefined data points 305, some significant outlier data points 301 may skew the entire LAT map 301, thereby degrading its diagnostic value.

While the LAT map of Fig. 2 may include many outliers, some of those that fall within the below 10% and above 90% percentiles are most probably the most significant. Therefore, highlighting the data points belonging to those percentiles, including outlier data points 303, makes it much easier for a user to remove the most significant outliers among these highlighted.

In another example, the processor highlights both the outlier data points belonging to the lowest percentile, and those belonging to the highest percentile, differently, which may make it even easier for a user to remove the most significant outliers among these highlighted.

In some examples, The processor highlights outlier data points that were detected based on one or more of the following outlier detection algorithms: Outliers Detection Using Inter Quartile Range (IQR), Z-score, Modified Z-score, Local Outliers Finder (LOF) and Density-Based Spatial Clustering for Application with Noise (DBSCAN).

In another example, the processor automatically rotates the map so that the physician can see the highlighted areas.

In the examples of Fig. 2, the EP values (e.g., LATs) of the data points are visualized using different grey levels drawn from a predefined greyscale. In real-life implementations, the EP values are typically represented using colors drawn from some color palette, although greyscale implementations are also feasible. In the context of the present disclosure, and in the claims, different grey levels are regarded as different colors, and references to colors and grey levels are used interchangeably.

### METHODS TO DISPLAY LOCATIONS OF EARLY AND LATE LAT IN LAT MAP AND TO CORRECT THE MAP

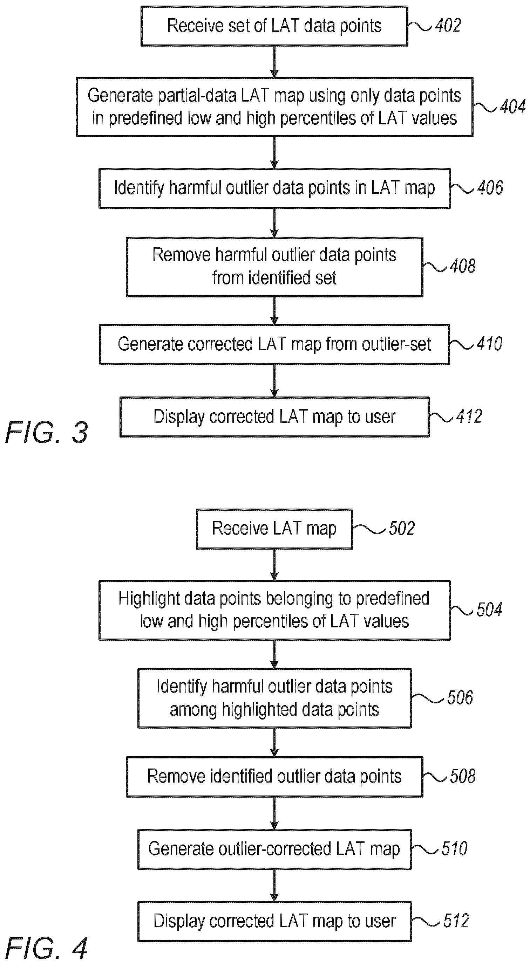

Fig. 3 is a flow chart that schematically illustrates a method for using a GUI 111 and a graphical input device 112 to correct an LAT map by removing outlier data points, in accordance with an example of the present disclosure. The process carries out an algorithm that begins with processor 56 receiving a set of LAT data points, at data receiving step 402.

Next, upon percentiles defined by a user (e.g., physician 24) and a command by physician 24 using GUI 111, the processor generates a partial-data LAT map based only on data points belonging to the selected percentiles, at a partial-data LAT map generation step 404.

At most significant outlier identification step 406, physician 24 identifies (e.g., selects) the most significant outliers on the partial-data LAT map.

In response to this selection, processor 56 removes the most significant outliers from the data set, at outlier removal step 408. The processor automatically generates a corrected LAT map from the outlier-corrected set, at LAT map regeneration step 410.

Finally, at an LAT map displaying step 412, processor 56 displays the corrected LAT map to physician 24.

The method described in Fig. 3 applies to any EP parameter having a range and outliers, such as activation voltage.

Fig. 4 is a flow chart that schematically illustrates a method for using a GUI 111 graphical input device 112 to correct an LAT map by removing outlier data points in accordance with another example of the present disclosure. The process carries out an algorithm that begins with processor 56 receiving an LAT map, at map receiving step 502.

Next, upon percentiles defined by a user (e.g., physician 24) and a command by physician 24 using GUI 111, the processor highlights only data points on the LAT map belonging to the selected percentiles, at a data point percentiles highlighting step 504.

At most significant outliers identification step 506, physician 24 identifies (e.g., selects) the most significant outliers on the highlighted LAT map.

In response to selection, processor 56 removes the most significant outliers from the data set used in generating the LAT map of step 502, at outlier removal step 508. The processor automatically generates a corrected LAT map from the outlier-corrected set, at LAT map regeneration step 510.

Finally, at an LAT map displaying step 512, processor 56 displays the corrected LAT map to physician 24.

The method described in Fig. 4 applies to any EP parameter having a range and outliers, such as activation voltage.

While the disclosed technique is mostly presented as applied to LAT maps, it can be, *mutatis mutandis,* applied to other types of EP maps, such as bipolar and unipolar voltage maps, or to any other EP map displaying an EP parameter having outliers within a given range of the EP parameter values.

### EXAMPLES

### Example 1

A system (10) includes a display device (27), an input device (112), and a processor (56). The processor (56) is configured to (i) receive a set of data points representing a full range of an EP parameter, (ii) responsively to one or more pre-defined percentile ranges, generate a partial-data EP map using only data points belonging to the one or more predefined percentile ranges, (iii) display the partial-data EP map to a user on the display device (27), (iv) receive from the user, via the input device (112), a selection on the partial-data EP map of one or more outlier data points belonging to one or more given percentile ranges, (v) regenerate the EP map using the set of data points without the selected outlier data points, and (vi) display the regenerated EP map to the user.

### Example 2

The system (10) according to example 1, wherein the processor (56) is further configured to provide, using the display device (27) and the input device (112), a graphical user interface (GUI) feature (111) that lets a user define the one or more percentile ranges of an Electrophysiological (EP) parameter.

### Example 3

The system (10) according to any of examples 1 and 2, wherein the GUI feature (111) is a button on the GUI that by pressing the button the processor (56) generates the partial-data EP map.

### Example 4

The system (10) according to any of examples 1 through 3, wherein the GUI feature (111) is further configured to highlight differently on the partial-data EP map data points belonging to different percentile ranges.

### Example 5

The system (10) according to example 1, wherein the processor (56) is further configured to apply one or more automatic outlier detection algorithms to highlight the outlier data points.

### Example 6

The system (10) according to any of examples 1 through 5, wherein the processor is further configured to rotate the map so that the physician can see the highlighted outlier data points.

### Example 7

The system (10) according to any of examples 1 through 6, wherein the input device (112) is further configured to allow the user to remove the outlier data points selected.

### Example 8

The system (10) according to any of examples 1 through 7, wherein the processor (56) is configured to automatically regenerate the EP map based on the set of data points after the selected outlier data points are removed from the set.

### Example 9

The system (10) according to any of examples 1 through 8, wherein the input device (112) comprises one of a touchscreen and a computer mouse.

### Example 10

The system (10) according to any of examples 1 through 9, wherein the EP parameter is local activation time (LAT).

### Example 11

The system (10) according to any of examples 1 through 10, wherein the EP map is an LAT map.

### Example 12

A system (10) includes a display device (27), an input device (112), and a processor (56). The processor (56) is configured to (i) present, on the display device (27), an EP map (301) having the full range of the EP parameter values, (ii) responsively to one or more predefined percentile ranges, highlight on the EP map data points belonging to the one or more given percentile ranges, and(iii) receive from the user, via the input device (112), a selection on the highlighted EP map (301) of one or more outlier data points (303) belonging to one or more given percentile ranges.

### Example 13

The system (10) according to example 12, wherein the processor (56) is further configured to apply one or more automatic outlier detection algorithms to highlight the outlier data points (303).

### Example 14

The system (10) according to any of examples 12 and 13, wherein the processor (56) is further configured to provide, using the display device (27) and the input device (112), a graphical user interface (GUI) feature (111) that lets a user define the one or more percentile ranges of an Electrophysiological (EP) parameter.

### Example 15

The system (10) according to example 13, wherein the GUI feature (111) is a virtual button on the GUI that by pressing the button the processor highlights the EP map.

### Example 16

The system according to any of examples 12 through 15, wherein the processor (56) is further configured to rotate the EP map so that the physician can see the highlighted data points.

### Example 17

The system (10) according to any of examples 12 through 18, wherein the processor (56) is configured to automatically regenerate the EP map (300) after the selected outlier data points (303) are removed from the set.

### Example 18

A method includes receiving a set of data points representing a full range of an EP parameter. Responsively to one or more pre-defined percentile ranges, a partial-data EP map is generated, using only data points belonging to the one or more predefined percentile ranges. The partial-data EP map is displayed to a user on a display device (27). A selection on the partial-data EP map of one or more outlier data points belonging to one or more given percentile ranges is received from the user, via an input device (112). The EP map is regenerated using the set of data points without the selected outlier data points. The regenerated EP map is displayed to the user.

### Example 19

A method includes presenting, on a display device (27), an EP map (301) having the full range of the EP parameter values. Responsively to one or more predefined percentile ranges, data points belonging to the one or more given percentile ranges are highlighted on the EP map (301). A selection on the highlighted EP map of one or more outlier data points (303) belonging to one or more given percentile ranges is received from the user, via an input device (112). The EP map is regenerated using the set of data points without the selected outlier data points. The regenerated EP map is displayed to the user.

### Example 20

A method, comprising: receiving a set of data points representing a full range of the EP parameter; responsively to one or more pre-defined percentile ranges, generating a partial-data EP map using only data points belonging to the one or more predefined percentile ranges; displaying the partial-data EP map to a user on a display device; receiving from the user, via an input device, a selection on the partial-data EP map of one or more outlier data points belonging to one or more given percentile ranges; regenerating the EP map using the set of data points without the selected outlier data points; and displaying the regenerated EP map to the user.

### Example 21

The method according to Example 20, and comprising providing, using the display device and the input device, a graphical user interface (GUI) feature that lets a user define the one or more percentile ranges of an Electrophysiological (EP) parameter.

### Example 22

The method according to Example 21, wherein the GUI feature is a button on the GUI that by pressing the button the partial-data EP map is generated.

### Example 23

The method according to Example 21, and comprising, using the GUI feature, highlighting differently on the partial-data EP map data points belonging to different percentile ranges.

### Example 24

The method according to Example 20, and comprising applying one or more automatic outlier detection algorithms to highlight the outlier data points.

### Example 25

The method according to Example 24, and comprising rotating the map so that the physician can see the highlighted outlier data points.

### Example 26

The method according to Example 20, and comprising, using the input device, allowing the user to remove the outlier data points selected.

### Example 27

The method according to Example 26, and comprising automatically regenerating the EP map based on the set of data points after the selected outlier data points are removed from the set.

### Example 28

The method according to Example 20, wherein the input device comprises one of a touchscreen and a computer mouse.

### Example 29

The method according to Example 20, wherein the EP parameter is local activation time (LAT).

### Example 30

The method according to Example 1 or Example 20, wherein the EP map is an LAT map.

### Example 31

A method, comprising: presenting on a display device, an EP map having the full range of the EP parameter values; responsively to one or more predefined percentile ranges, highlighting on the EP map data points belonging to the one or more given percentile ranges; receiving from the user, via an input device, a selection on the highlighted EP map of one or more outlier data points belonging to one or more given percentile ranges; regenerating the EP map using the set of data points without the selected outlier data points; and displaying the regenerated EP map to the user.

### Example 32

The method according to Example 31, and comprising applying one or more automatic outlier detection algorithms to highlight the outlier data points.

### Example 33

The method according to Example 31, and comprising providing, using the display device and the input device, a graphical user interface (GUI) feature that lets a user define the one or more percentile ranges of an Electrophysiological (EP) parameter.

### Example 34

The method according to Example 33, wherein the GUI feature is a button on the GUI that by pressing the button the processor highlights the EP map.

### Example 35

The method according to Example 33, and comprising, using the GUI feature, highlighting differently on the partial-data EP map data points belonging to different percentile ranges.

### Example 36

The method according to Example 31, and comprising rotating the map so that the physician can see the highlighted data points.

### Example 37

The method according to Example 31, and comprising, using the input device, allowing the user to remove the outlier data points selected.

### Example 38

The method according to Example 31, and comprising automatically regenerating the EP map after the selected outlier data points are removed from the set.

### Example 39

The method according to Example 31, wherein the input device comprises one of a touchscreen and a computer mouse.

### Example 40

The method according to Example 31, wherein the EP parameter is LAT and the EP map is an LAT map.

Although the examples described herein mainly address cardiac diagnostic applications, the methods and systems described herein can also be used in other medical applications.

It will be appreciated that the examples described above are cited by way of example, and that the present disclosure is not limited to what has been particularly shown and described hereinabove. Rather, the scope of the present disclosure includes both combinations and subcombinations of the various features described hereinabove, as well as variations and modifications thereof which would occur to persons skilled in the art upon reading the foregoing description and which are not disclosed in the prior art.

## Claims

1. A system (10), comprising:
a display device (27);
an input device (112); and
a processor (56), configured to:
receive a set of data points representing a full range of an EP parameter;
responsively to one or more pre-defined percentile ranges, generate a partial-data EP map using only data points belonging to the one or more predefined percentile ranges;
display the partial-data EP map to a user on the display device (27);
receive from the user, via the input device (112), a selection on the partial-data EP map of one or more outlier data points belonging to one or more given percentile ranges;
regenerate the EP map using the set of data points without the selected outlier data points; and
display the regenerated EP map to the user.

2. The system (10) according to claim 1, wherein the EP parameter is local activation time, LAT and/or the EP map is an LAT map.

3. A system (10), comprising:
a display device (27);
an input device (112); and
a processor (56), configured to:
present, on the display device (27), an EP map (301) having the full range of the EP parameter values;
responsively to one or more predefined percentile ranges, highlight on the EP map data points belonging to the one or more given percentile ranges;
receive from the user, via the input device (112), a selection on the highlighted EP map (301)of one or more outlier data points (303) belonging to one or more given percentile ranges;
regenerate the EP map using the set of data points without the selected outlier data points; and
display the regenerated EP map to the user.

4. The system (10) according to claim 1 or claim 3, wherein the processor (56) is further configured to apply one or more automatic outlier detection algorithms to highlight the outlier data points (303).

5. The system (10) according to claim 1 or claim 3, wherein the processor (56) is further configured to provide, using the display device (27) and the input device (112), a graphical user interface, GUI, feature (111) that lets a user define the one or more percentile ranges of an Electrophysiological, EP parameter.

6. The system (10) according to claim 5 when dependent on claim 1, wherein the GUI feature (111) is a button on the GUI that by pressing the button the processor (56) generates the partial-data EP map.

7. The system (10) according to claim 5 when dependent on claim 1, wherein the GUI feature (111) is further configured to highlight differently on the partial-data EP map data points belonging to different percentile ranges.

8. The system (10) according to claim 5 when dependent on claim 3, wherein the GUI feature (111) is a button on the GUI that by pressing the button the processor highlights the EP map.

9. The system (10) according to claim 5 when dependent on claim 3, wherein the GUI feature (111) is further configured to highlight differently on the EP map data points belonging to different percentile ranges.

10. The system (10) according to claim 3 or claim 4 when dependent on claim 1, wherein the processor (56) is further configured to rotate the map so that the physician can see the highlighted data points.

11. The system (10) according to claim 1 or claim 3, wherein the input device (112) is further configured to allow the user to remove the outlier data points selected.

12. The system (10) according to claim 11 when dependent on claim 1, wherein the processor (56) is configured to automatically regenerate the EP map based on the set of data points after the selected outlier data points are removed from the set.

13. The system (10) according to claim 11 when dependent on claim 3, wherein the processor (56) is configured to automatically regenerate the EP map after the selected outlier data points are removed from the set.

14. The system (10) according to claim 1 or claim 3, wherein the input device (112) comprises one of a touchscreen and a computer mouse.

15. The system (10) according to claim 3, wherein the EP parameter is LAT and the EP map is an LAT map.
